# EUROPÄISCHE PATENTSCHRIFT

(11) **EP 1 478 338 B1**
(45) Veröffentlichungstag und Bekanntmachung des Hinweises auf die Patenterteilung: **06.07.2005**
(21) Anmeldenummer: 03706518.2
(22) Anmeldetag: 17.02.2003
(51) Int. Cl.: A61K 9/00

(54) **PULVERINHALATIVUM, ENTHALTEND DEN CGRP-ANTAGONISTEN BIBN4096 UND VERFAHREN ZU DESSEN HERSTELLUNG**
POWDER INHALATION CONTAINING CGRP-ANTAGONIST BIBN4096 AND METHOD FOR THE PRODUCTION THEREOF
POUDRE D'INHALATION CONTENANT L'ANTAGONISTE BIBN4096 DE CGRP ET PROCEDE DE FABRICATION

(30) Priorität: 20.02.2002 DE 10207026
(43) Veröffentlichungstag der Anmeldung: 24.11.2004
(73) Patentinhaber: Boehringer Ingelheim Pharma GmbH & Co.KG, 55216 Ingelheim am Rhein (DE)
(72) Erfinder: TRUNK, Michael, 55218 Ingelheim am Rhein (DE); WEILER, Claudius, 55218 Ingelheim-Grosswinterheim (DE)
(86) Internationale Anmeldenummer: PCT/EP2003/001563
(87) Internationale Veröffentlichungsnummer: WO 2003/070215

(56) Entgegenhaltungen:
- WO-A-01/10425
- WO-A-01/32144
- DE-A- 1 792 207
- DE-A- 10 139 410

## Beschreibung

Die Erfindung betrifft ein Pulverinhalativum umfassend den CGRP-Antagonisten 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin [BIBN4096] der Formel I in Form sphärisch nanostrukturierter Mikropartikel, die unter Normalbedingungen (T < 50°C, relative Feuchte < 75%) in ihrem amorphen Zustand stabil vorliegen sowie ein Verfahren zu dessen Herstellung, mit Hilfe dessen der thermodynamisch stabile bzw. stabilisierte Wirkstoff im amorphen Zustand in einem einzigen Schritt zu Mikropartikeln verarbeitet werden kann.

Die erfindungsgemäßen sphärisch nanostrukturierten Mikropartikel eignen sich zur Herstellung von Pulverinhalativa, wobei keine weiteren Hilfsstoffe oder Zuschlagsstoffe (Trägermaterialien) benötigt werden, um ein technisch handhabbares Pulver, das sich direkt weiterverarbeiten lässt und das ausgezeichnete Eigenschaften hinsichtlich der Dispergierbarkeit aufweist und bezüglich seiner kohäsiven Eigenschaften ausreichend gut verarbeitbar ist, zu erhalten. Ein weiterer Aspekt der Erfindung sind die mittels des erfindungsgemäßen Verfahrens erhältlichen Pulverinhalativa.

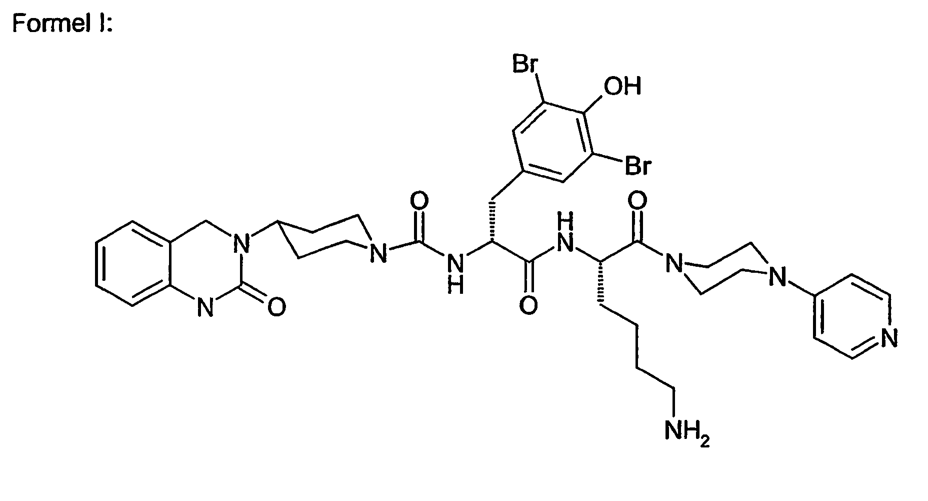

### Stand der Technik

BIBN4096 stellt einen hochwirksamen CGRP-Antagonisten zur Behandlung von Migräne dar, dessen Applikation mittels klassischer Darreichungsformen nicht auf oralem Wege möglich ist, da die Substanz oral nur eine geringe Bioverfügbarkeit aufweist.

Bei der Applikationsform Pulverinhalativa werden Inhalationspulver, die in geeignete Kapseln (Inhaletten) abgefüllt werden, mittels Pulverinhalatoren in der Lunge ausgebracht. Alternativ dazu kann eine inhalative Anwendung auch durch Applikation geeigneter pulverförmiger Inhalationsaerosole, die beispielsweise ein HFA134a, HFA227 oder deren Gemisch als Treibgas enthalten, erfolgen.
Dabei werden die Mikropartikel des reinen Wirkstoffs durch die Atemwege auf der Lungenoberfläche z.B. in den Alveolen mittels des Inhalationsvorganges appliziert. Diese Partikel sedimentieren auf der Oberfläche und können erst nach dem Lösevorgang durch aktive und passive Transportvorgänge im Körper aufgenommen werden.

Bekannt in der Literatur sind Inhalationssysteme, in denen der Wirkstoff entweder als mikronisierte Suspension in einem passenden Lösungsmittelsystem als Träger vorliegt oder in Form eines trockenen Pulvers.
Üblicherweise werden Pulverinhalativa z.B. in Form von Kapseln zur Inhalation auf Basis der allgemeinen Lehre, wie sie in DE-A-179 22 07 beschrieben ist, unter Verwendung der chemisch stabilsten Form des Wirkstoffs hergestellt. Dabei werden pharmazeutische Präparate, hergestellt durch Mischen eines fein zerteilten Medikaments mit einem gröberen Trägermedium, in einem Luftstrom durch ein sogenanntes "Staubfließverfahren" unter Ausnutzung der Saugfunktion des Inhalators als Hauptenergiequelle dispergiert.

Ein kritischer Faktor bei solchen Mehrstoffsystemen ist eine gleichmäßige Verteilung des Arzneimittels in der Pulvermischung. Weiterhin ist eine zusätzliche Belastung der Lunge durch den Trägerstoff gegeben sowie das Auftreten von unerwünschten Wechselwirkungen, was zu Kompatibilitätsproblemen führen kann.

Ein bedeutender Aspekt bei der inhalativen Applikation des Wirkstoffes ist, dass nur Teilchen einer bestimmten aerodynamischen Größe in das Zielorgan Lunge gelangen. Die Teilchengröße dieser lungengängigen Partikel (inhalierbarer Anteil) liegt im Submikronbereich. Solche Partikel werden üblicherweise durch Mikronisierung (Luftstrahlmahlung) erzeugt. Daraus ergibt sich oftmals, dass solche Partikel durch diesen mechanischen Schritt hinsichtlich ihrer Kristalleigenschaften komplex zusammengesetzt sein können. Ebenso bedingt die geometrische Form der Partikel des Ausgangsmaterials die morphologischen Eigenschaften des Mikronisats.

Neben dem Strahlmahlverfahren, wobei dem Luftstrahlmahlverfahren eine besondere Bedeutung zukommt, ist es auch möglich, ein geeignetes Mikronisat über Alternativverfahren herzustellen. Geeignete Mikronisierverfahren zur Herstellung von Mikropartikeln im Submikronbereich sind z.B. das Fällungsverfahren einschließlich der Verfahren, bei denen der Wirkstoff durch Einengen des Lösungsmittels über die maximale Löslichkeit hinaus als nicht-kristalliner Feststoff (amorph) abgeschieden werden kann, die Fällung mittels superkritischer Gase, wie z.B. das RESS- oder das PGSS-Verfahren (J. Jung, M. Perrut: *Particle Design Using Supercritical Fluids, J.* Supercrit. Fluids 20 (2001), 179-219), das GASR-Verfahren (M.P. Gallager et al.: *Gas Antisolvent Recrystallization,* Am. Chem. Soc. (1989)), das PCA-Verfahren (D.J. Dixon, K.P. Johnston: *Polymeric Materials Formed by Precipitation with compressed Fluid Antisolvent*, AIChE Journal (1993, Vol. 39(1), 127), die Gefriertrocknung, die Sprühtrocknung oder eine Kombination aus mehreren der vorstehend genannten Verfahren.

Literaturbekannt ist, dass mittels Sprühtrocknung lungengängige Partikel mit einer Größe zwischen 0.5 µm und 10 µm, bevorzugt zwischen 0.5 µm und 6 µm, hergestellt werden können. Üblicherweise werden aus solchen Sprühtrocknungspartikeln in Anlehnung an oben zitiertes Verfahren (DE-A-179 22 07) technisch handhabbare Formulierungen hergestellt, die eine ausreichende Dispergierbarkeit bei der medizinischen Anwendung (Inhalation) aufweisen [Y.-F. Maa, P.-A. Ngyuyen, J.D. Andya, N. Dasovich, T.D. Sweeny, S.J. Shire, C.C. Hsu, Pharmaceutical Research, 15, No. 5 (1998), 768-775; M.T. Vidgrén, P.A. Vidgrén, T.P. Paronen, Int. J. Pharmaceutics, 35 (1987), 139-144; R.W. Niven, F.D. Lott, A.Y. Ip, J.M. Cribbs, Pharmaceutical Research, 11, No. 8 (1994), 1101-1109].

Neben diesen Beispielen gibt es weitere, vor allem von pharmazeutischen Unternehmen vorgestellte Herstelltechniken auf Basis von Sprühtrocknungsverfahren, die spezielle Formulierungen für Pulverinhalativa beschreiben.

Neben den vorstehend angegebenen Erfordernissen ist generell zu berücksichtigen, dass jede Änderung des Feststoffzustandes eines Arzneimittels, welche dessen physikalische und chemische Stabilität sowie technischen Eigenschaften verbessern kann, gegenüber weniger stabilen Formen desselben Arzneimittels einen erheblichen Vorteil ergibt.

### Problemstellung

Die komplexe Aufgabe der vorliegenden Erfindung bestand primär in der Bereitstellung einer bioverfügbaren Formulierung für den hochwirksamen CGRP-Antagonisten BIBN4096. Die erfindungsgemäße Formulierung sollte zur Behandlung der akuten, bei Migräne sehr plötzlich eintretenden Schmerzzustände einen schnellen Wirkungseintritt zeigen. Das bedeutet, dass eine rasche Aufnahme des Wirkstoffs und ein schneller Anstieg des Plasmaspiegels gewährleistet sein müssen.

### Beschreibung der Erfindung

Ein schneller Wirkungseintritt zur Behandlung von akuten Schmerzzuständen sowie ein hoher Plasmaspiegel des Wirkstoffs BIBN4096 innerhalb kürzester Zeit läßt sich neben der intravenösen Gabe am besten über die Lunge als Aufnahmeorgan realisieren.

Im Rahmen der vorliegenden Erfindung wurde nun überraschend gefunden, dass BIBN4096 in Form der Wirkstoffbase durch eine inhalative Darreichung in ausreichendem Maße bioverfügbar gemacht werden kann. Es hat sich herausgestellt, dass bei inhalativer Gabe des Wirkstoffs in Form sphärisch nanostrukturierter Mikropartikel eine Bioverfügbarkeit von ca. 60% bezogen auf den Feinanteil der Formulierung (entspricht FPD bestimmt nach USP 24 Suppl. 2000) erreicht werden kann.

Die erfindungsgemäße Formulierung benötigt keinen Zusatz von Trägermaterialien.

Ein erster Gegenstand der vorliegenden Erfindung ist somit ein Pulverinhalativum, umfassend die Wirkstoffbase 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin [BIBN4096] der Formel (I) in Form sphärisch nanostrukturierter Partikel, dadurch gekennzeichnet, dass
(a) die Partikel eine spezifische Oberfläche zwischen 1 m²/g und 25 m²/g, bevorzugt zwischen 1 m²/g und 20 m²/g, besonders bevorzugt zwischen 3 m²/g und 10 m²/g, aufweisen
(b) der Kennwert Q_{(5.8)} zwischen 50% und 100% und
(c) der Parameter X₅₀ zwischen 1 µm und 6 µm liegt.

Diese Mikropartikel zeichnen sich durch spezielle physikalische und physikochemische Eigenschaften aus, die zu einer verbesserten pharmakologischen/pharmakokinetischen Wirkung in der inhalativen Anwendung der Substanz führen. Die Verfügbarkeit der Substanz -sowohl quantitativ, bezogen auf die verabreichte Wirkstoffmenge, als auch bezogen auf einen möglichst schnell zu erreichenden hohen Plasmaspiegel- wird neben den biochemischen Eigenschaften der Substanz auch durch physikochemische Eigenschaften bedingt. Sofern ein Feststoff -wie im Falle eines Pulverinhalativums- verabreicht wird, sind im speziellen hierbei die Parameter absolute Löslichkeit im Umgebungsmedium als auch Lösungsgeschwindigkeit im Umgebungsmedium als Funktion der lokalen Konzentration des Wirkstoffes und der Zeit zu berücksichtigen.
Eine optimale inhalative Darreichung muss daher berücksichtigen, dass die Wirkstoffpartikel fein verteilt die Lungenoberfläche belegen. Ausschlaggebend dafür ist, dass der Wirkstoff so verändert wird, dass die zu inhalierenden Mirkopartikel hinsichtlich ihrer Partikel-Partikel-Wechselwirkung sowie ihren Dispersions- bzw. aerodynamischen Eigenschaften Vorteile aufweisen, die bedingen, dass diese einerseits quantitativ in den tieferen Lungenbereich abgelagert werden und andererseits eine möglichst große Lungenoberfläche belegt wird. Daher sind die physikalischchemischen Eigenschaften der zu inhalierenden Mikropartikel für Pulverinhalativa von großer Bedeutung.

Die nach dem erfindungsgemäßen Verfahren dargestellten Partikel weisen eine hohe physikalische Stabilität auf. Insbesondere ermöglichen die Partikeleigenschaften bei der Anwendung als Pulverinhalativum einen hohen Feinanteil bei der Ausbringung, technisch bestimmt z.B. mittels Kaskadenimpaktormessung (Andersenkaskaden-Impaktor, nach USP 24 bzw. Pharm. Eur. Suppl. 2000). Typischerweise ist der Anteil der Partikel nach dieser Methode, die kleiner als 5 µm (aerodynamisch) sind, größer als 15%, es werden dabei z.T. sogar Feinanteile von mehr als 50% erreicht. Neben diesem Key-Parameter für Inhalativa zeichnet sich das Pulver dadurch aus, dass es sich mit gängigen technischen Verfahren weiterverarbeiten lässt. Gekennzeichnet sind so hergestellte Pulver durch die physikochemischen Parameter Partikelgröße, z.B. gemessen mittels Laserbeugung, sowie spezifische Oberfläche, z.B. gemessen mittels Mehrpunkt B.E.T. Messung. Für den Kennwert Q_{(5.8)} liegt die Partikelgröße so hergestellter Pulver typischerweise zwischen 50% und 100% sowie für den Parameter X₅₀ zwischen 1 µm und 6 µm. Partikel, die nach obigen Verfahren hergestellt sind, weisen dabei typischerweise Werte für die Spezifische Oberfläche zwischen 1 m²/g und 25 m²/g, idealerweise zwischen 1 m²/g und 20 m²/g und in besonders bevorzugter Weise zwischen 3 m²/g und 10 m²/g auf. Geometrisch weisen Partikel, die nach obigen Verfahren hergestellt werden, Partikelformen auf, die je nach Versuchsbedingung zwischen den Extrema "Kugelform", "Kugelform mit Hohlraum, evtl. mit Loch", "Kugelform mit nach innen-geformten Wölbungen", sowie "zusammengefallene Hohlkörper" beschrieben werden können. Rasterelektronenmikroskopisch ist die Oberfläche solcher Partikel weitestgehend nanostrukturiert.

Erfindungsgemäß wurde gefunden, dass BIBN4096 in Form der freien Base durch ein Sprühtrocknungsverfahren überraschenderweise morphologisch so verändert werden kann, dass ein derart hergestelltes Pulver ohne weitere Schritte, vor allem ohne die Notwendigkeit des Mischens mit einem gröberen Trägermaterial, direkt in ein Primärpackmittel abgefüllt werden kann und aus diesem heraus mittels eines Pulverinhalationsdevices zur Inhalation ausgebracht werden kann.

Dabei kann das Herstellverfahren so gesteuert werden, dass die Partikel in geeigneter Korngröße vorliegen, üblicherweise zwischen 0.1 und 10 µm und diese Partikel solche Oberflächeneigenschaften besitzen, dass diese leicht verwirbelbar/dispergierbar sind.

Es wurde ferner gefunden, dass die Partikelmorphologie einschließlich der Partikelgröße maßgeblich durch die Wahl der Prozeßparameter und Herstellparameter gezielt gesteuert werden kann. Dabei ist es überraschend, dass Pulver dieser Substanz, die mittels "klassischem" Strahlmahlverfahren mikronisiert wurden und in einem vergleichbaren Korngrößenspektrum vorliegen, sich jedoch morphologisch von Partikeln, die gemäß dieser Erfindung hergestellt wurden, bezüglich ihrer Oberflächeneigenschaften/Partikel-Partikel-Wechselwirkungen grundsätzlich unterscheiden. Dies zeigt sich daran, dass sich der Qualitätsparameter "Fine Particle Fraction of Delivered Dose" (z.B. nach Methode zur Bestimmung der "Aerodynamic Particle Size Distribution" - USP 24 oder Pharm. Eur. Suppl. 2000) um den Faktor 10 und mehr verbessert. Da gleichzeitig auf ein Trägermaterial in der Formulierung verzichtet werden kann, verbessert sich bei vorgegebener applizierbarer Pulvergesamtmenge die tatsächlich dem Patienten verfügbare absolute Wirkstoffdosis um einen noch bedeutend höheren Faktor.

Das erfindungsgemäße Herstellverfahren ist dadurch gekennzeichnet, dass der Wirkstoff in geeigneter Weise gelöst, versprüht und in einem Sprühturm getrocknet wird. Das Prinzip der Sprühtrocknung besteht darin, eine Lösung oder Suspension des zu trocknenden Produkts in feine Tröpfchen zu zerteilen und mit einem heißen Gasstrom zu trocknen. Der nach Verdampfen des Lösungsmittels zurückbleibende Feststoffanteil wird aus dem Gasstrom mittels eines Massenkraftabscheiders (z.B. Zyklon) und/oder durch eine Filtereinheit abgetrennt und gesammelt. Die so hergestellten Mikropartikel zeichnen sich dabei durch spezielle Werte hinsichtlich Partikelgröße, spezifischer Oberfläche und Morphologie aus.

Als geeignete Lösungsmittel haben sich organische Lösungsmittel bzw. organischwässrige Lösungsmittelgemische herausgestellt. Vorzugsweise verwendet man ein alkoholisch-wäßriges Lösungsmittelsystem, besonders bevorzugt ein Lösungsmittelgemisch bestehend aus Ethanol/Methanol/Wasser sowie Ethanol/Propanol/Wasser und ganz besonders bevorzugt das Lösungsmittelgemisch Ethanol/Wasser. Hierbei ist der Molanteil des Wassers in den Lösungsmittelgemischen von der 0.1-fachen bis zur 10-fachen Menge des Molanteils der Alkoholkomponenten, in bevorzugter Weise von der 0.5-fachen bis zur 4-fachen Menge, einzusetzen.
Die Einstellung der Wirkstoffkonzentration dient primär dazu, den Prozess wirtschaftlich zu gestalten. Dabei sind jedoch der einzustellenden Wirkstoffkonzentration Grenzen gesetzt, die dadurch vorgegeben werden, dass die Oberflächeneigenschaften der Partikel durch ein bestimmtes Verhältnis zwischen Tropfengröße und Feststoffkonzentration optimiert werden können. Üblicherweise ist eine Konzentration zwischen 0.5 und 20 Gewichtsprozent, in bevorzugter Art und Weise zwischen 2 und 10 Gewichtsprozent, in sehr bevorzugter Art und Weise zwischen 3 und 8 Gewichtsprozent zu wählen. Die Tropfengröße ist ein entscheidender Parameter zur Erzeugung inhalierbarer Partikel. In Abhängigkeit der verwendeten Düse ist der Sprühgasdurchsatz in Kombination mit dem Lösungsdurchsatz so zu wählen, dass die gewünschte Tropfengröße erzielt wird. Da es eine Vielzahl von Parameter-Kombinationen Düse-Sprühgasdurchsatz-Lösungsdurchsatz gibt, die zu einer geeigneten Tropfengröße führen, wird eine sinnvolle Spezifizierung des Verfahrens über die Tropfengröße getroffen, welche beim Prozess gewählt werden soll. Diese kann durch den Kennwert X₅₀ (Medianwert = Teilchengröße/Tropfengröße, unterhalb derer 50% der Teilchenmenge liegt bezüglich der Volumenverteilung der einzelnen Teilchen/Tropfen), der im Bereich zwischen 1.5 µm und 20 µm, bevorzugt zwischen 1.5 µm und 8 µm, liegen sollte, sowie den Kennwert Q_{(5.8)} (entspricht der Teilchenmenge, die bezogen auf die Volumenverteilung der Tröpfchen unterhalb von 5.8 µm liegt), der zwischen 10% und 100%, bevorzugt zwischen 30% und 100%, liegen sollte, charakterisiert werden.

Technisch umgesetzt wird dies, in dem eine entsprechende kommerzielle Düse, z. B. Ein- oder Mehrstoffdüsen, die in Abhängigkeit der Düsenparameter (z.B. Rotationsgeschwindigkeit bei Rotationszerstäubern oder angesetztem Zerstäubungsdruck und des daraus resultierenden Massestroms des Zerstäubungsgases bei Zweistoffdüsen) sowie der Spray-Rate (Volumenstrom "Sprühlösung") diese Charakteristika aufweist, eingesetzt wird. Neben den besonderen Bedingungen, die im eigentlichen Sprühprozess eingehalten werden müssen, um geeignete Tröpfchen für den Trocknungsprozess zu generieren, zeigt sich, dass die Oberflächeneigenschaften der Partikel auch durch die Wahl der Trocknungsparameter positiv/gezielt beeinflusst werden können. Die entscheidenden Kenngrößen, die in den Trocknungsschritt einfließen, sind Eingangs- und Ausgangstemperatur des Trocknungsgases, sowie der Volumenstrom des durchgesetzten Trockengases. Es ist zu beachten, dass die Tropfen mit geeigneter Tropfengröße so durch die Trocknungskammer geführt werden, dass die Tröpfchen und die getrockneten Partikel nicht oder nur geringfügig in Berührung mit der Wand des Sprühturms kommen. Dies wird durch Düsen mit entsprechendem Sprühkegel, durch einen Sprühturm mit geeignetem Durchmesser und durch die Strömungsbedingungen in der Apparatur erzielt. Die Ausgangstemperatur muss für den Prozess so angepasst werden, dass das Pulver einen ausreichend geringen Restlösemittelgehalt aufweist und somit eine ausreichende chemische und physikalische Stabilität erreicht wird. Diese ist idealerweise gegeben, wenn die Ausgangs-Temperatur im Bereich der Siedetemperatur bzw. gering darüber gehalten wird. Dagegen ist die Einlasstemperatur des Trocknungsgases so zu wählen, dass in Kombination mit dem Parameter Volumenstrom "Trocknungsgas" sowie Spray-Rate das Trocknen so schonend abläuft, dass Partikel mit geeigneten Oberflächeneigenschaften entstehen.

Ein zweiter Gegenstand der Erfindung ist somit ein Verfahren zur Herstellung der Wirkstoffbase BIBN4096 in Form sphärisch nanostrukturierter Mikropartikel, umfassend die Schritte
(a) Lösen des Wirkstoffs BIBN4096 in einem organischen Lösungsmittel oder einem organisch-wässrigen Lösungsmittelgemisch zur Herstellung einer Lösung des Wirkstoffs mit einer Wirkstoffkonzentration zwischen 0.5 und 20 Gew.-%, bevorzugt zwischen 2 und 10 Gew.-%, besonders bevorzugt zwischen 3 und 8 Gew.-%,
(b) Versprühen der so gewonnenen Wirkstofflösung auf übliche Weise, so dass ein Sprühnebel mit einer Tropfengröße mit dem Kennwert X₅₀ im Bereich von 1.5 bis 20 µm, bevorzugt im Bereich von 1.5 bis 8 µm, und Q_{(5.8)} zwischen 10 und 100 %, bevorzugt zwischen 30 und 100%, erzielt wird,
(c) Trocknen des so erhaltenen Sprühnebels mit Hilfe eines Trocknungsgases unter Anwendung folgender Parameter:
   - einer Eingangstemperatur des Trocknungsgases von 100 °C bis 350 °C, bevorzugt zwischen 120 °C und 250 °C und besonders bevorzugt zwischen 130 °C und 200 °C,
   - einer Ausgangstemperatur des Trocknungsgases von 40 °C bis 120 °C,
   - einem Volumenstrom des Sprühgases von 1 Nm³/h bis 15 Nm³/h und
   - einem Volumenstrom des Trocknungsgases von 15 Nm³/h bis 1500 Nm³/h, bevorzugt zwischen 15 Nm³/h bis 150 Nm³/h, und
(d) Abtrennen des getrockneten Feststoffanteils aus dem Trockungsgasstrom auf übliche Weise.

Ein dritter Gegenstand der Erfindung ist die Verwendung der Wirkstoffbase BIBN4096 in Form von sphärisch nanostrukturierten Mikropartikeln, erhältlich nach dem vorstehend beschriebenen Verfahren, zur Herstellung eines Pulverinhalativums.

Ein vierter Gegenstand der vorliegenden Erfindung ist ein Pulverinhalativum, dadurch gekennzeichnet, dass die sphärisch nanostrukturierten Partikel gemäß dem voranstehend genannten erfindungsgemäßen Verfahren erhältlich sind.

### Experimenteller Teil

### 1) Meßverfahren

### a) Bestimmung der Partikelgröße mittels Laserbeugung (Frauenhoferbeugung):

- Meßmethode:: Zur Bestimmung der Partikelgröße wird das Pulver mittels Dispergiereinheit einem Laserbeugungs-Spektrometer zugeführt. Unter dem Medianwert X₅₀ versteht man die Teilchengröße, unterhalb derer 50% der Teilchenmenge liegt. Der Q_{(5,8)} - Wert beschreibt den prozentualen Anteil der Teilchen, die eine Größe unterhalb von 5.8 µm aufweisen.
- Meßgerät:: Laser-Beugungs-Spektrometer (HELOS),Fa. Sympatec
- Software:: WINDOX Version 3.3/REL 1 für Beispiele 1 bis 3 und Version 4 für Beispiele 4 bis 6
- Dispergiereinheit:: RODOS / Dispergierdruck: 3 bar
- Brennweite:: 100 mm [Meßbereich: 0.9.....175 µm]
- Auswertemodus:: HRLD (V 3.3 Rel. 1)

### b) Bestimmung der Spezifischen Oberfläche:

- Meßmethode:: Die Bestimmung der spezifischen Oberfläche erfolgt, indem die Pulverprobe einer Stickstoffatmosphäre bei unterschiedlichen Drücken ausgesetzt wird. Durch Abkühlung der Probe erfolgt eine Kondensation der Stickstoffmolekülen auf der Oberfläche der Partikel. Die kondensierte Stickstoffmenge wird über den Druckabfall im System bestimmt und die spezifische Oberfläche der Probe über den Flächenbedarf von Stickstoff und der Probeneinwaage berechnet.
- Meßgerät: Tri Star Multi Point BET, Fa. Micromeritics
- Ausheizstation:: VacPrep 061, Fa. Micromeritics
- Ausheizen:: ca. 12h / 40°C

| Analysenparameter | |
|---|---|
| Probengefäß | ½ inch; mit "filler rod" |
| Analyseverfahren | 16 Punkt BET Oberflächenbestimmung 0.05 bis 0.20 p/p0 |
| absolute Drucktoleranz | 5.0 mm Hg |
| relative Drucktoleranz | 5.0% |
| Evakuierungs-Geschwindigkeit | 50.0 mm Hg/Sekunde |
| Evakuierungsschwellenwert | 10.0 mm Hg |
| Evakuierungsdauer | 0.1 h |
| Leervolumen | Dewargefäß-Absenkung, t: 0.5 h |
| Haltezeit | 20 Sekunden |
| Minimale Gleichgewichts-einstellungsdauer | 600 Sekunden |
| Adsorbens | Stickstoff |

### c) Bestimmung der Tropfengröße mittels Laserbeugung (nach Mie):

- Meßgerät:: Laser-Beugungs-Spektrometer (HELOS), Fa. Sympatec
- Software:: WINDOX Version 4
- Brennweite:: 100 mm [Meßbereich: 0.9.....175 µm]
- Meßmethode:: Die Ermittlung der Tropfengröße erfolgt, indem die Düse aus dem Sprühtrockner herausgenommen wird und der Spray im oberen Drittels des Sprühkegels zentrisch in den Laserstrahl gebracht wird. Die Messung erfolgt bei Raumtemperatur mit Wasser als Referenzmedium unter ansonsten gleichen Bedingungen.

### 2) Beispiele

### Beispiel 1: Sprühparameter, geeignet für eine alkoholische BIBN4096-Lösung (modifizierter BÜCHI-Sprühtrockner):

| | | |
|---|---|---|
| Konzentration Lösung/ | | 7 g BIBN 4096 in 100 ml |
| Zusammensetzung Lösungsmittel | | Ethanol/Methanol/H₂O |
| | | molares Verhältnis: 1 : 1 : 2.3 |
| Tröpfchengröße | Q_{(5.8)} | 46% (Auswertung nach Mie); |
| (Referenzlösung: H₂O bei Raumtemperatur) | | 51 % (Auswertung nach Frauenhofer) |
| | X₅₀ | |
| | | 6.3 µm (Auswertung nach Mie); |
| | | 5.7 µm (Auswertung nach Frauenhofer) |
| Volumenstrom "Sprührate" | | 18 ml / min |
| Sprühdruck (*Düsentyp*) | | 2.9 bar Überdruck (N₂) |
| | | *(BÜCHI Sprühdüse 0.7 mm,* |
| | | *Art.-Nr. 04364)* |
| Volumenstrom "Zerstäubungsdruck" (Düsentyp) | | 1775 Normliter / h |
| | | *(BÜCHI Sprühdüse 0.7 mm,* |
| | | *Art.-Nr. 04364)* |
| Eingangstemperatur | | 150 °C |
| Ausgangstemperatur | | 100 °C |
| Volumenstrom "Trocknungsgas" | | 30 Norm m³ / h |
| Querschnitt Trockenturm | | 105 mm |

### Charakterisierung der erhaltenen Feststoffpartikel:

| | | |
|---|---|---|
| Partikelgröße | X₅₀ | 1.9 µm |
| | Q_{(5.8)} | 98.3% |

### Beispiel 2: Sprühparameter, geeignet für eine alkoholische BIBN4096-Lösung (modifizierter BÜCHI-Sprühtrockner):

| | | |
|---|---|---|
| Konzentration Lösung/ | | 7 g BIBN 4096 in 100 ml |
| Zusammensetzung Lösungsmittel | | Ethanol/Methanol/H₂O |
| | | molares Verhältnis: 1 : 1 : 2.3 |
| Tröpfchengröße | Q_{(5 8)} | 27% (Auswertung nach Mie) |
| (Referenzlösung: H₂O bei Raumtemperatur) | | 39% (Auswertung nach Frauenhofer) |
| | X₅₀ | |
| | | 8.9 µm (Auswertung nach Mie) |
| | | 7.3 µm (Auswertung nach Frauenhofer) |
| Volumenstrom "Sprührate" | | 18 ml / min |
| Sprühdruck (*Düsentyp*) | | 2.9 bar Überdruck (N₂) |
| | | *(BÜCHI Sprühdüse 0.7 mm,* |
| | | *Art.-Nr. 04364)* |
| Volumenstrom "Zerstäubungsdruck" (Düsentyp) | | 1482 Normliter / h |
| | | *(BÜCHI Sprühdüse 0.7 mm,* |
| | | *Art.-Nr. 04364)* |
| Eingangstemperatur | | 150 °C |
| Ausgangstemperatur | | 100 °C |
| Volumenstrom "Trocknungsgas" | | 30 Norm m³ / h |
| Querschnitt Trockenturm | | 105 mm |

### Charakterisierung der erhaltenen Feststoffpartikel:

| | | |
|---|---|---|
| Partikelgröße | X₅₀ | 2.4 µm |
| | Q_{(5.8)} | 87% |

### Beispiel 3: Sprühparameter, geeignet für eine alkoholische BIBN4096-Lösung (modifizierter BÜCHI-Sprühtrockner):

| | | |
|---|---|---|
| Konzentration Lösung/ | | 7.4 g BIBN 4096 in 100 g |
| Zusammensetzung Lösungsmittel | | Ethanol/Methanol/H₂O |
| | | molares Verhältnis: 1 : 1 : 2.3 |
| Tröpfchengröße | Q_{(5.8)} | < 10% |
| (Referenzlösung: H₂O bei Raumtemperatur) | | |
| | X₅₀ | 17 µm |
| Volumenstrom "Sprührate" | | 1.04 l / h |
| Sprühdruck *(Düsentyp)* | | 0.8 bar Überdruck (N₂) |
| | | *(BÜCHI Sprühdüse 0.7 mm,* |
| | | *Art.-Nr. 04364)* |
| Volumenstrom "Zerstäubungsdruck" (Düsentyp) | | 0.6 kg / h |
| | | *(BÜCHI Sprühdüse 0.7 mm,* |
| | | *Art.-Nr. 04364)* |
| Eingangstemperatur | | 150 °C |
| Ausgangstemperatur | | 100 °C |
| Volumenstrom "Trocknungsgas" | | 35-36 Norm m³ / h |
| Querschnitt Trockenturm | | 105 mm |

### Charakterisierung der erhaltenen Feststoffpartikel:

| | | |
|---|---|---|
| Partikelgröße | X₅₀ | 5.7 µm |
| | Q_{(5.8)} | 50.7% |
| Spezifische Oberfläche | Sₘ | 19.6 m²/g |

### Beispiel 4: Sprühparameter, geeignet für eine alkoholische BIBN4096-Lösung (modifizierter BÜCHI-Sprühtrockner):

| | | |
|---|---|---|
| Konzentration Lösung/ | | 7.0 g BIBN 4096 in 100 g |
| Zusammensetzung Lösungsmittel | | Ethanol/Methanol/H₂O |
| | | molares Verhältnis: 1 : 1 : 2.3 |
| Tröpfchengröße | Q_{(5.8)} | 59% |
| (Referenzlösung: H₂O bei Raumtemperatur) | | |
| | X₅₀ | 6.5 µm |
| Volumenstrom "Sprührate" | | 1.08 l / h |
| Sprühdruck (*Düsentyp*) | | 5.5 bar Überdruck (N₂) |
| | | *(BÜCHI Sprühdüse 0.7 mm,* |
| | | *Art.-Nr. 04364)* |
| Volumenstrom "Zerstäubungsdruck" | | 3.4 kg / h |
| (Düsentyp) | | *(BÜCHI Sprühdüse 0.7 mm,* |
| | | *Art.-Nr. 04364)* |
| Eingangstemperatur | | 150 °C |
| Ausgangstemperatur | | 95 °C |
| Volumenstrom "Trocknungsgas" | | 36 Norm m³ / h |
| Querschnitt Trockenturm | | 105 mm |

### Charakterisierung der erhaltenen Feststoffpartikel:

| | | |
|---|---|---|
| Partikelgröße | X₅₀ | 1.4 µm |
| | Q_{(5.8)} | 99.7% |
| Spezifische Oberfläche | Sₘ | 7.3 m²/g |

### Beispiel 5: Sprühparameter, geeignet für eine alkoholische BIBN4096-Lösung (modifizierter BÜCHI-Sprühtrockner):

| | | |
|---|---|---|
| Konzentration Lösung/ Zusammensetzung Lösungsmittel | | 9.9 g BIBN 4096 in 100 g Ethanol/H₂O molares Verhältnis: 2 : 3 |
| Tröpfchengröße | Q_{(5.8)} | 59% |
| (Referenzlösung: H₂O bei Raumtemperatur) | | |
| | X₅₀ | 6.5 µm |
| Volumenstrom "Sprührate" | | 1.2 l / h |
| Sprühdruck *(Düsentyp)* | | 5.4 bar Überdruck (N₂) |
| | | *(BÜCHI Sprühdüse 0. 7 mm,* |
| | | *Art.-Nr. 04364)* |
| Volumenstrom "Zerstäubungsdruck" (Düsentyp) | | 3.4 kg / h |
| | | *(BÜCHI Sprühdüse 0. 7 mm,* |
| | | *Art.-Nr. 04364)* |
| Eingangstemperatur | | 150 °C |
| Ausgangstemperatur | | 101 °C |
| Volumenstrom "Trocknungsgas" | | 36 Norm m³ / h |
| Querschnitt Trockenturm | | 105 mm |

### Charakterisierung der erhaltenen Feststoffpartikel:

| | | |
|---|---|---|
| Partikelgröße | X₅₀ | 2.7 µm |
| | Q_{(5.8)} | 90.0% |
| Spezifische Oberfläche | Sₘ | 5.7 m²/g |

### Beispiel 6: Sprühparameter, geeignet für eine alkoholische BIBN4096-Lösung (modifizierter BÜCHI-Sprühtrockner):

| | | |
|---|---|---|
| Konzentration Lösung/ Zusammensetzung Lösungsmittel | | 4.0 g BIBN 4096 in 100 g Ethanol/H₂O molares Verhältnis: 2 : 3 |
| Tröpfchengröße | Q_{(5.8)} | 59% |
| (Referenzlösung: H₂O bei Raumtemperatur) | | |
| | X₅₀ | 6.5 µm |
| Volumenstrom "Sprührate" | | 1.2 l / h |
| Sprühdruck *(Düsentyp)* | | 5.5 bar Überdruck (N₂) |
| | | *(BÜCHI Sprühdüse 0. 7 mm,* |
| | | *Art.-Nr. 04364)* |
| Volumenstrom "Zerstäubungsdruck" (Düsentyp) | | 3.4 kg / h |
| | | *(BÜCHI Sprühdüse 0.7 mm,* |
| | | *Art.-Nr. 04364)* |
| Eingangstemperatur | | 150 °C |
| Ausgangstemperatur | | 100 °C |
| Volumenstrom "Trocknungsgas" | | 36 Norm m³ / h |
| Querschnitt Trockenturm | | 105 mm |

### Charakterisierung der erhaltenen Feststoffpartikel:

| | | |
|---|---|---|
| Partikelgröße | X₅₀ | 1.5 µm |
| | Q_{(5.8)} | 99.7% |
| Spezifische Oberfläche | Sₘ | 7.5 m²/g |

### Kurzbeschreibung der Abbildung

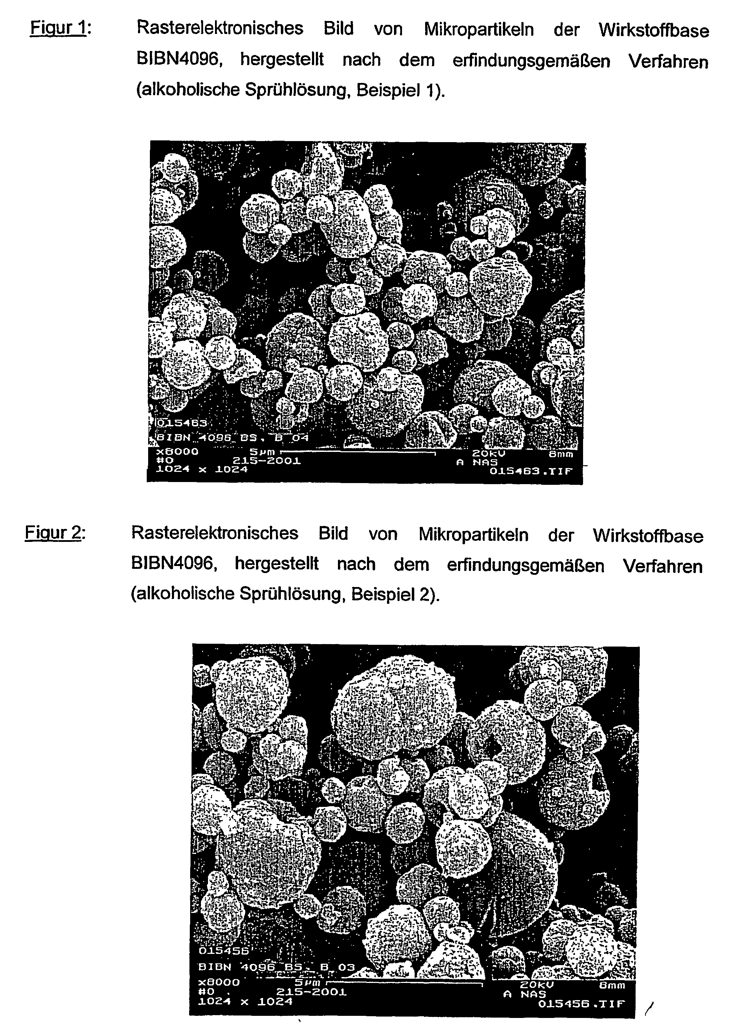

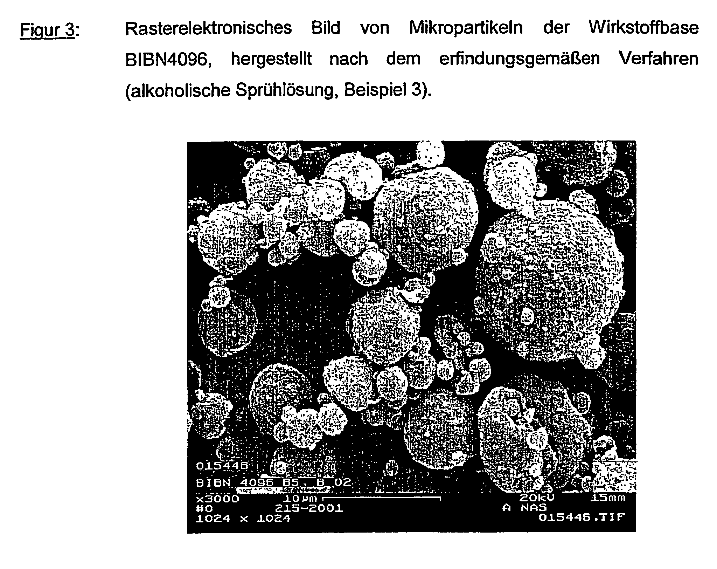

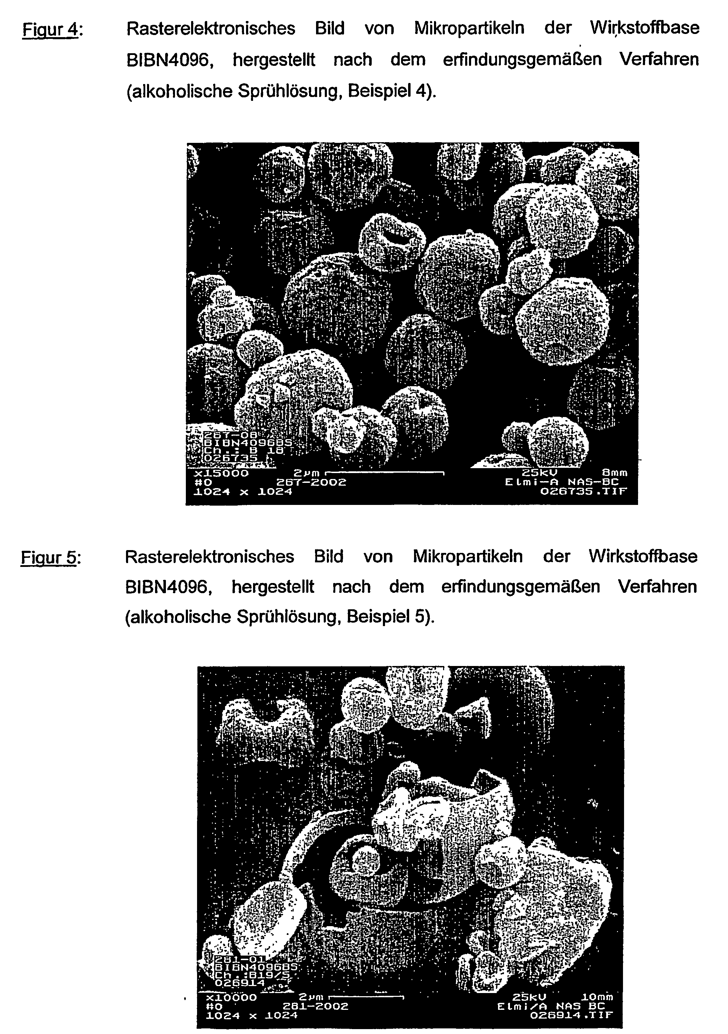

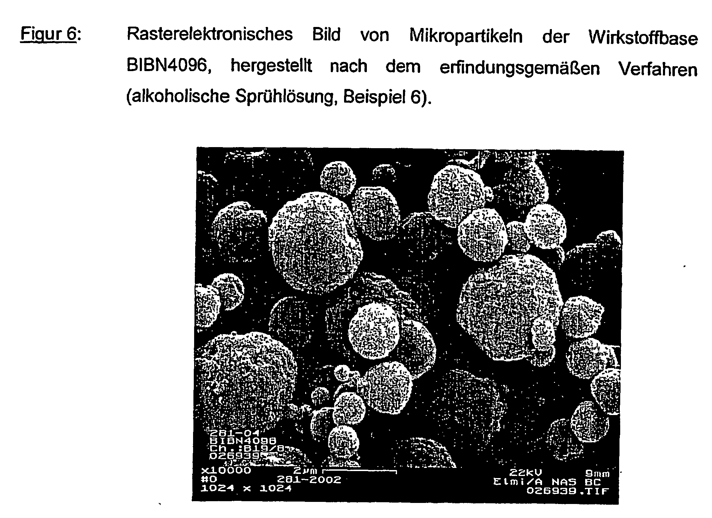

Die Figuren 1 bis 6 zeigen die Aufnahmen von Mikropartikeln der Wirkstoffbase BIBN4096, die nach dem erfindungsgemäßen Verfahren aus einer alkoholischen Sprühlösung hergestellt wurden.

## Patentansprüche

1. Pulverinhalativum, umfassend die Wirkstoffbase 1-[N²-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin [BIBN4096] der Formel 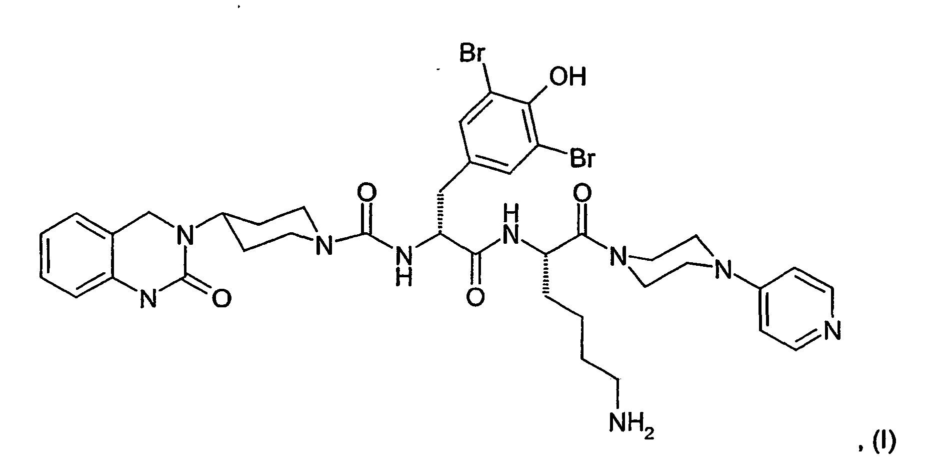 in Form sphärisch nanostrukturierter Partikel, **dadurch gekennzeichnet, dass**
(a) die Partikel eine spezifische Oberfläche zwischen 1 m²/g und 25 m²/g aufweisen,
(b) der Kennwert Q_{(5.8)} zwischen 50% und 100% und
(c) der Parameter X₅₀ zwischen 1 µm und 6 µm liegt.

2. Pulverinhalativum gemäß Anspruch 1, **dadurch gekennzeichnet, dass** die Partikel eine spezifische Oberfläche zwischen 1 m²/g und 20 m²/g aufweisen.

3. Pulverinhalativum gemäß Anspruch 1, **dadurch gekennzeichnet, dass** die Partikel eine spezifische Oberfläche zwischen 3 m²/g und 10 m²/g aufweisen.

4. Verfahren zur Herstellung der Wirkstoffbase BIBN4096 in Form sphärisch nanostrukturierter Mikropartikel, umfassend die Schritte
(a) Lösen des Wirkstoffs BIBN4096 in einem organischen Lösungsmittel oder einem organisch-wässrigen Lösungsmittelgemisch zur Herstellung einer Lösung des Wirkstoffs mit einer Wirkstoffkonzentration zwischen 0.5 und 20 Gew.-%,
(b) Versprühen der so gewonnenen Wirkstofflösung auf übliche Weise, so dass ein Sprühnebel mit einer Tropfengröße mit dem Kennwert X₅₀ im Bereich von 1.5 bis 20 µm, bevorzugt im Bereich von 1.5 µm bis 8 µm, und Q_{(5.8)} zwischen 10 und 100%, bevorzugt zwischen 30 und 100%, erzielt wird,
(c) Trocknen des so erhaltenen Sprühnebels mit Hilfe eines Trocknungsgases unter Anwendung folgender Parameter:
• einer Eingangstemperatur des Trocknungsgases von 100 °C bis 350 °C, bevorzugt zwischen 120 °C und 250 °C und besonders bevorzugt zwischen 130 °C und 200 °C,
• einer Ausgangstemperatur des Trocknungsgases von 40 °C bis 120 °C,
• einem Volumenstrom des Sprühgases von 1 Nm³/h bis 15 Nm³/h und
• einem Volumenstrom des Trocknungsgases von 15 Nm³/h bis 1500 Nm³/h, bevorzugt von 15 Nm³/h bis 150 Nm³/h, und
(d) Abtrennen des getrockneten Feststoffanteils aus dem Trockungsgasstrom auf übliche Weise.

5. Verfahren nach Anspruch 4, **dadurch gekennzeichnet, dass** das zum Lösen des Wirkstoffs verwendete Lösungsmittel ein organisch-wäßriges Lösungsmittelsystem ist, wobei der Molanteil des Wassers von 0.1 bis zur 10-fachen Menge des Molanteils der Alkoholkomponenten, in bevorzugter Weise von 0.5 bis zur 4-fachen Menge, einzusetzen ist.

6. Verfahren nach Anspruch 4, **dadurch gekennzeichnet, dass** das organischwäßrige Lösungsmittelsystem aus Ethanol/Methanol/Wasser besteht, wobei der Molanteil des Wassers von 0.1 bis zur 10-fachen Menge des Molanteils der Alkoholkomponenten, in bevorzugter Weise von 0.5 bis zur 4-fachen Menge, einzusetzen ist.

7. Verfahren nach Anspruch 4, **dadurch gekennzeichnet, dass** das organischwäßrige Lösungsmittelsystem aus Ethanol/Propanol/Wasser besteht, wobei der Molanteil des Wassers von 0.1 bis zur 10-fachen Menge des Molanteils der Alkoholkomponenten, in bevorzugter Weise von 0.5 bis zur 4-fachen Menge, einzusetzen ist.

8. Verfahren nach Anspruch 4, **dadurch gekennzeichnet, dass** das organischwäßrige Lösungsmittelsystem aus Ethanol/Wasser besteht, wobei der Molanteil des Wassers von 0.1 bis zur 10-fachen Menge des Molanteils der Alkoholkomponente, in bevorzugter Weise von 0.5 bis zur 4-fachen Menge, einzusetzen ist.

9. Verfahren nach einem der Ansprüche 4 bis 8, **dadurch gekennzeichnet, dass** die zur Sprühtrocknung verwendete Lösung des Wirkstoffs eine Konzentration von 2 bis 10 Gew. % hat.

10. Verfahren nach einem der Ansprüche 4 bis 8, **dadurch gekennzeichnet, dass** die zur Sprühtrocknung verwendete Lösung des Wirkstoffs eine Konzentration von 3 bis 8 Gew. % hat.

11. Verwendung der Wirkstoffbase BIBN4096 in Form der sphärisch nanostrukturierten Mikropartikel, erhältlich gemäß einem der Ansprüche 4 bis 10, zur Herstellung eines Pulverinhalativums.

12. Pulverinhalativum gemäß einem der Ansprüche 1 bis 3, **dadurch gekennzeichnet, dass** die sphärisch nanostrukturierten Partikel nach einem Verfahren gemäß einem der Ansprüche 4 bis 10 erhältlich sind.

## Claims

1. Inhalation powder containing the active substance base 1-[N²-[3,5-dibromo-N-[[4-(3,4-dihydro-2(1H)-oxoquinazolin-3-yl)-1-piperidinyl]carbonyl]-D-tyrosyl]-L-lysyl]-4-(4-pyridinyl)-piperazine [BIBN4096] of formula 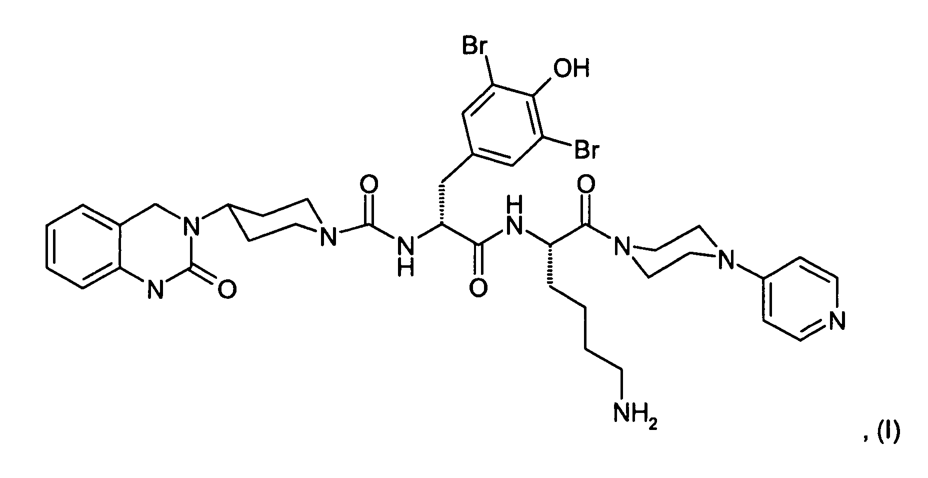 in the form of spherically nanostructured particles, **characterised in that**
(a) the particles have a specific surface area of between 1 m²/g and 25 m²/g,
(b) the characteristic value Q_{(5.8)} is between 50% and 100% and
(c) the parameter X₅₀ is between 1 µm and 6 µm.

2. Inhalation powder according to claim 1, **characterised in that** the particles have a specific surface of between 1 m²/g and 20 m²/g.

3. Inhalation powder according to claim 1, **characterised in that** the particles have a specific surface of between 3 m²/g and 10 m²/g.

4. Process for preparing the active substance base BIBN4096 in the form of spherically nanostructured microparticles, comprising the steps of
a) dissolving the active substance BIBN4096 in an organic solvent or an organic-aqueous solvent mixture to prepare a solution of the active substance with an active substance concentration of between 0.5 and 20 percent by weight,
b) spraying the resulting solution of active substance in conventional manner so as to obtain a spray mist with a droplet size having the characteristic value X₅₀ in the range from 1.5 to 20 µm, preferably in the range from 1.5 to 8 µm, and Q_{(5.8)} between 10% and 100%, preferably between 30% and 100%,
c) drying the spray mist thus obtained by means of a drying gas, while applying the following parameters:
• an inlet temperature of the drying gas of 100°C to 350°C, preferably between 120 °C and 250 °C and more preferably between 130 °C and 200°C,
• an outlet temperature of the drying gas of 40 °C to 120 °C,
• a volumetric flow of the spray gas of 1 Nm³/h to 15 Nm³/h,
• a volumetric flow of the drying gas of 15 Nm³/h to 1500 Nm³/h, preferably 15 Nm³/h to 150 Nm³/h, and
d) separating the dried solid fraction from the drying gas current in conventional manner.

5. Process according to claim 4, **characterised in that** the solvent used to dissolve the active substance is an organic aqueous solvent system wherein the molar proportion of water to be used is from 0.1 to 10 times the molar proportion of the alcohol components, preferably from 0.5 to 4 times as much.

6. Process according to claim 4, **characterised in that** the organic-aqueous solvent system consists of ethanol/methanol/water, wherein the molar proportion of water to be used is from 0.1 to 10 times the molar proportion of the alcohol components, preferably from 0.5 to 4 times as much.

7. Process according to claim 4, **characterised in that** the organic-aqueous solvent system consists of ethanol/propanol/water, wherein the molar proportion of water to be used is from 0.1 to 10 times the molar proportion of the alcohol components, preferably from 0.5 to 4 times as much.

8. Process according to claim 4, **characterised in that** the organic-aqueous solvent system consists of ethanol/water, wherein the molar proportion of water to be used is from 0.1 to 10 times the molar proportion of the alcohol component, preferably from 0.5 to 4 times as much.

9. Process according to one of claims 4 to 8, **characterised in that** the solution of the active substance used for spray-drying has a concentration of 2 to 10 percent by weight.

10. Process according to one of claims 4 to 8, **characterised in that** the solution of the active substance used for spray-drying has a concentration of 3 to 8 percent by weight.

11. Use of the active substance base BIBN4096 in the form of the spherically nanostructured microparticles obtainable according to one of claims 4 to 10, for the manufacture of an inhalable powder.

12. Inhalation powder according to one of claims 1 to 3, **characterised in that** the spherically nanostructured particles may be obtained by a process according to one of claims 4 to 10.

## Revendications

1. Produit pour inhalation pulvérulent comprenant la base de principe actif 1-[N²-[3,5-dibromo-N-[[4-(3,4-dihydro-2(1H)-oxoquinazolin-3-yl)-1-pipéridinyl]carbonyl]-D-tyroxyl]-L-lysyl]-4-(4-pyridinyl)-pipérazine [BIBN4096] de formule 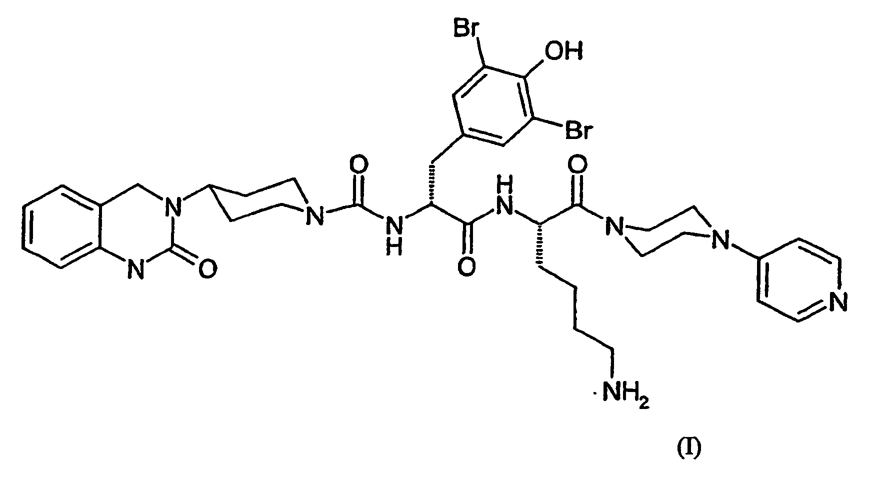 sous forme de particules nanostructurées sphériquement, **caractérisé en ce que**
(a) les particules présentent une surface spécifique entre 1 m²/g et 25 m²/g,
(b) la valeur caractéristique Q_{(5.8)} est située entre 50 % et 100 % et
(c) le paramètre X₅₀ est situé entre 1 µm et 6 µm.

2. Produit pour inhalation pulvérulent selon la revendication 1 **caractérisé en ce que** les particules présentent une surface spécifique entre 1 m²/g et 20 m²/g.

3. Produit pour inhalation pulvérulent selon la revendication 1 **caractérisé en ce que** les particules présentent une surface spécifique entre 3 m²/g et 10 m²/g.

4. Procédé de production de la base de principe actif BIBN4096 sous forme de microparticules nanostructurées sphériquement, comprenant les étapes
(a) dissolution du principe actif BIBN4096 dans un solvant organique ou un mélange de solvants organo-aqueux pour la production d'une solution du principe actif ayant une concentration de principe actif entre 0,5 et 20 % en masse,
(b) pulvérisation de la solution de principe actif ainsi obtenue de la manière habituelle, de sorte qu'un brouillard de pulvérisation ayant une taille de gouttes avec la valeur caractéristique X₅₀ dans le domaine de 1,5 à 20 µm, de préférence dans le domaine de 1,5 µm à 8 µm, et Q_{(5.8)} entre 10 et 100 %, de préférence entre 30 et 100 %, est obtenu,
(c) séchage du brouillard de pulvérisation ainsi obtenu à l'aide d'un gaz de séchage au moyen des paramètres suivants :
- une température d'entrée du gaz de séchage de 100°C à 350°C, de préférence entre 120°C et 250°C et de manière particulièrement préférée entre 130°C et 200°C,
- une température de sortie du gaz de séchage de 40°C à 120°C,
- un courant volumique du gaz de pulvérisation de 1 Nm³/h à 15 Nm³/h et
- un courant volumique du gaz de séchage de 15 Nm³/h à 1500 Nm³/h, de préférence de 15 Nm³/h à 150 Nm³/h, et
(d) séparation de la portion de solide séchée d'avec le courant de gaz de séchage de la manière habituelle.

5. Procédé selon la revendication 4 **caractérisé en ce que** le solvant utilisé pour la dissolution du principe actif est un système de solvant organo-aqueux où la portion molaire de l'eau de 0,1 à 10 fois la quantité de la portion molaire du composant alcoolique, de préférence de 0,5 à 4 fois la quantité, doit être utilisée.

6. Procédé selon la revendication 4 **caractérisé en ce que** le système de solvant organo-aqueux consiste en éthanol/méthanol/eau où la portion molaire de l'eau de 0,1 à 10 fois la quantité de la portion molaire du composant alcoolique, de préférence de 0,5 à 4 fois la quantité, doit être utilisée.

7. Procédé selon la revendication 4 **caractérisé en ce que** le système de solvant organo-aqueux consiste en éthanol/propanol/eau où la portion molaire de l'eau de 0,1 à 10 fois la quantité de la portion molaire du composant alcoolique, de préférence de 0,5 à 4 fois la quantité, doit être utilisée.

8. Procédé selon la revendication 4 **caractérisé en ce que** le système de solvant organo-aqueux consiste en éthanol/eau où la portion molaire de l'eau de 0,1 à 10 fois la quantité de la portion molaire du composant alcoolique, de préférence de 0,5 à 4 fois la quantité, doit être utilisée.

9. Procédé selon l'une des revendications 4 à 8 **caractérisé en ce que** la solution du principe actif utilisée pour le séchage par pulvérisation a une concentration de 2 à 10 % en masse.

10. Procédé selon l'une des revendications 4 à 8 **caractérisé en ce que** la solution du principe actif utilisée pour le séchage par pulvérisation a une concentration de 3 à 8 % en masse.

11. Utilisation de la base de principe actif BIBN4096 sous forme des microparticules nanostructurées sphériquement, pouvant être obtenue selon l'une des revendications 4 à 10, pour la production d'un produit pour inhalation pulvérulent.

12. Produit pour inhalation pulvérulent selon l'une des revendications 1 à 3 **caractérisé en ce que** les particules nanostructurées sphériquement peuvent être obtenues selon un procédé selon l'une des revendications 4 à 10.
